(19) Europäisches Patentamt / European Patent Office / Office européen des brevets

(11) **EP 4 516 329 A1**

(12) **EUROPEAN PATENT APPLICATION**
published in accordance with Art. 153(4) EPC

(43) Date of publication:
**05.03.2025 Bulletin 2025/10**

(21) Application number: **23796500.9**

(22) Date of filing: **27.04.2023**

(51) International Patent Classification (IPC):
**A61L 27/16** (2006.01)   **A61L 27/20** (2006.01)
**A61L 27/52** (2006.01)

(52) Cooperative Patent Classification (CPC):
**A61L 27/16; A61L 27/20; A61L 27/52**

(86) International application number:
**PCT/JP2023/016713**

(87) International publication number:
**WO 2023/210765 (02.11.2023 Gazette 2023/44)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.04.2022 JP 2022075173**

(71) Applicants:
• **Kuraray Co., Ltd.**
  **Kurashiki-shi, Okayama 710-0801 (JP)**
• **Kuraray Kuraflex Co., Ltd.**
  **Okayama 702-8045 (JP)**

(72) Inventors:
• **KOBAYASHI, Goro**
  **Tsukuba-shi, Ibaraki 305-0841 (JP)**
• **AYANO, Satoru**
  **Tsukuba-shi, Ibaraki 305-0841 (JP)**
• **FUJITA, Akio**
  **Tsukuba-shi, Ibaraki 305-0841 (JP)**
• **MATSUSHITA, Kazuhiro**
  **Okayama-shi, Okayama 702-8045 (JP)**
• **TOMOI, Masanori**
  **Saijo-shi, Ehime 793-8585 (JP)**

(74) Representative: **Müller-Boré & Partner**
**Patentanwälte PartG mbB**
**Friedenheimer Brücke 21**
**80639 München (DE)**

(54) **IMMUNOISOLATION DEVICE**

(57)    An object of the present invention is to provide an immunoisolation device that achieves both a reduction in diffusion distance, which is effective in improving the permeability of substances, such as physiologically active substances and nutrients, and an improvement in durability to withstand long-term transplantation, and that further comprises a cell-capturing layer capable of preventing the escape of cells or a cell cluster. The present invention provides an immunoisolation device having a cell-capturing layer (A) and an immunoisolation layer (B) that coveres the cell-capturing layer (A), the cell-capturing layer (A) comprising a fiber structure (a1).

Fig. 3A

EP 4 516 329 A1

## Description

Technical Field

The present invention relates to an immunoisolation device.

Background Art

Immunoisolation devices have been developed as a means for performing cell transplantation therapy without the need to administer an immunosuppressant. In particular, for example, in transplantation of somatic cells derived from iPS cells, for which the risk of canceration is a concern, or at the time of a decrease in the function of transplanted cells, macroencapsulation immunoisolation devices are considered an effective method in that the transplantation site can be identified and that the devices can be replaced. Important functions required of macroencapsulation immunoisolation devices are the following: cells or cell clusters can be uniformly dispersed and fixed without incurring association of cells or cell clusters; the devices allow for easy permeation of oxygen and nutritional components to transplanted cells; the devices allow for easy release of a desired physiologically active substance (cytokines, hormones, growth factors, etc.) from cells, which is required for a therapeutic effect, according to cell response; permeation of immunoresponsive cells and immune response factors is prevented; and the transplanted devices are excellent in biocompatibility, and are less likely to adhere to surrounding tissue and less likely to induce inflammatory responses, such as granulation.

Many immunoisolation devices using porous membranes have been studied (Patent Literature (PTL) 1) and have been considered to have one problem, that is a decrease in permeability due to protein adsorption onto porous membrane materials and/or fibrosis, or a decrease in permeability due to adhesion to surrounding tissue.

Internal necrosis of cell clusters or the like is considered to be induced when the diameter exceeds 500 um. In order to easily maintain engraftment of embedded cells to a recipient and release physiologically active substances, it is necessary to appropriately control the overall thickness of such an immunoisolation device, and it is desirable that the immunoisolation layer constituting the device is as thin as possible. On the other hand, for transplantation into the body, it is necessary to improve the membrane strength and durability to prevent the device from breaking or being twisted. However, it is not easy to achieve both a reduction in membrane thickness and improvement in durability.

Citation List

Patent Literature

PTL 1: JP2012-508584A

Summary of Invention

Technical Problem

An object of the present invention is to provide an immunoisolation device that is highly durable in long-term transplantation and that is suitable for improving the diffusion efficiency of physiologically active substances required for transplantation while maintaining immunoisolation effects.

Another object of the present invention is to provide an invention that achieves both a reduction in diffusion distance in the device and improvement in durability, while having excellent biocompatibility and maintaining immunoisolation properties without interfering with the engraftment of the transplanted material, such as cells and cell clusters.

Further, another object of the present invention is to provide an immunoisolation device comprising a cell-capturing layer capable of preventing escape of cells or a cell cluster.

Solution to Problem

The present invention provides the following immunoisolation devices.

[1] An immunoisolation device comprising

a cell-capturing layer (A) and
an immunoisolation layer (B) that covers the cell-capturing layer (A),
the cell-capturing layer (A) comprising a fiber structure (a1).

[2] The immunoisolation device according to [1], wherein the fiber structure (a1) has a porosity of 90% or more.

[3] The immunoisolation device according to [1] or [2], wherein the fiber structure (a1) has a thickness of 100 to 2000 μm.

[4] The immunoisolation device according to any one of [1] to [3], wherein the fiber structure (a1) comprises at least one member selected from the group consisting of ethylene-vinyl alcohol copolymers and cellulose acetate.

[5] The immunoisolation device according to any one of [1] to [4], wherein

> the cell-capturing layer (A) comprises the fiber structure (a1) and a dense fiber structure (a2), and
> the dense fiber structure (a2) is disposed around the fiber structure (a1) and has an average pore size of 35 μm or less.

[6] The immunoisolation device according to [5], wherein the dense fiber structure (a2) has a thickness of 1500 μm or less.

[7] The immunoisolation device according to [5] or [6], wherein the dense fiber structure (a2) comprises at least one member selected from the group consisting of ethylene-vinyl alcohol copolymers and cellulose acetate.

[8] The immunoisolation device according to [1], wherein

> the cell-capturing layer (A) comprises a dense fiber structure (a2) disposed around the fiber structure (a1) and is capable of preventing escape of a cell or a cell cluster captured in the fiber structure (a1), and
> the dense fiber structure (a2) has the function of preventing escape of the cells or the cell cluster.

[9] The immunoisolation device according to any one of [1] to [8], wherein

> the immunoisolation layer (B) comprises a porous membrane (b1) or a fiber structure (b2), and
> the porous membrane (b1) or the fiber structure (b2) comprises at least one member selected from the group consisting of ethylene-vinyl alcohol copolymers and cellulose acetate.

[10] The immunoisolation device according to [9], wherein the immunoisolation layer (B) is an immunoisolation multilayer (B') comprising the porous membrane (b1) or the fiber structure (b2), and a hydrogel (b3).

[11] The immunoisolation device according to [10], wherein the immunoisolation multilayer (B') has a thickness of 500 μm or less.

[12] The immunoisolation device according to [10] or [11], wherein the outermost layer of the immunoisolation multilayer (B') is composed of the porous membrane (b1) or the fiber structure (b2), and the innermost layer is composed of the hydrogel (b3).

[13] The immunoisolation device according to [10] or [11], wherein the outermost layer of the immunoisolation multilayer (B') is composed of the hydrogel (b3) and the innermost layer is composed of the porous membrane (b1) or the fiber structure (b2).

[14] The immunoisolation device according to any one of [10] to [13], wherein the hydrogel (b3) comprises a polyvinyl alcohol and the average degree of polymerization of the polyvinyl alcohol is 300 to 10000.

[15] A method for producing an immunoisolation device comprising a cell-capturing layer (A) and an immunoisolation multilayer (B'),

> the cell-capturing layer (A) comprising a fiber structure (a1), and
> the immunoisolation multilayer covering the cell-capturing layer (A),
> the method comprising the steps of:

> > (1) applying a hydrosol solution to a porous membrane (b1);
> > (2) converting the hydrosol solution into a hydrogel by application of heat, temperature, light, or chemical action to form an immunoisolation multilayer (B'); and
> > (3) molding the immunoisolation multilayer (B') into a pouch-like shape by heat fusion.

[16] The method for producing an immunoisolation device according to [15], wherein the hydrosol solution in step (1) comprises a polyvinyl alcohol,

> the polyvinyl alcohol has a degree of polymerization of 300 to 10000, and
> the hydrosol solution has a solids concentration of 3 to 15 mass%.

[17] The method for producing an immunoisolation device according to [15] or [16], wherein

the heat fusion in step (3) is performed with a resin being interposed between two sheets of the immunoisolation multilayer (B'), and the two sheets of the immunoisolation multilayer (B') are disposed with their hydrogel surfaces facing each other, and

the resin comprises at least one member selected from the group consisting of ethylene-vinyl alcohol copolymers and cellulose acetate.

Effect of the Invention

The present invention can provide an immunoisolation device that achieves both a reduction in diffusion distance, which is effective in improving the permeability of substances, such as physiologically active substances and nutrients, and an improvement in durability to withstand long-term transplantation, the immunoisolation device having excellent biocompatibility and further comprising a cell-capturing layer capable of preventing the escape of cells or a cell cluster.

Brief Description of Drawings

Fig. 1 is a perspective view of a pouch-like immunoisolation device.
Fig. 2 is a cross-sectional view of a tubular immunoisolation device.
Fig. 3A is a conceptual diagram of an immunoisolation device using a fiber structure (nonwoven fabric) as a cell-capturing layer.
Fig. 3B is a conceptual diagram of an immunoisolation device using a fiber structure (nonwoven fabric) as a cell-capturing layer.
Fig. 4 is a conceptual diagram of a cell-capturing layer.
Fig. 5 is a transmitted light microscopy image of a dense fiber structure (a2).
Fig. 6 is a cross-sectional view of an immunoisolation layer having a two-layer structure consisting of a porous membrane and a hydrogel.
Fig. 7 is a cross-sectional view of an immunoisolation multilayer formed of a nonwoven fabric and a hydrogel.
Fig. 8 is an SEM image of a cross-section of the immunoisolation layer (B) obtained in Production Example 1.
Fig 9 is an outline of a material permeability test.

Description of Embodiments

The use of the singular forms ("a," "an," and "the") in the present specification shall be construed to include both the singular and the plural unless the context clearly indicates that only the singular is intended.
In this specification, the term "comprise" is a concept that encompasses "consist essentially of" and "consist only of."
The immunoisolation device of the present invention comprises a cell-capturing layer (A) capable of capturing cells, which are a material to be transplanted, and an immunoisolation layer (B), wherein the cell-capturing layer (A) is covered with the immunoisolation layer (B) to thereby inhibit the invasion of immune cells and cytokines into the cell-capturing layer (A). The cell-capturing layer (A) comprises a fiber structure (a1). The immunoisolation layer (B) is preferably composed of a porous membrane (b1) or fiber structure (b2), and a hydrogel (b3).

Cell-capturing Layer (A)

Fiber Structure (a1)

The fiber structure (a1) contained in the cell-capturing layer (A) functions as a scaffold for capturing cells or cell clusters, which are a material to be transplanted. The fiber structure of the immunoisolation layer (a1) of the cell-capturing layer (A) is, for example, a non-woven fabric, a woven fabric, or a knitted fabric, and is preferably a non-woven fabric.
The material of the fiber structure (a1) preferably contains at least one member selected from the group consisting of ethylene-vinyl alcohol copolymers and cellulose acetate, and more preferably contains an ethylene-vinyl alcohol copolymer. Such materials may be used alone to form the fiber structure (a1), or two or more of them may be used in a combination to form the fiber structure (a1). The fiber structure (a1) may be a cell scaffold material, such as collagen fibers.
The ethylene-vinyl alcohol copolymer can be usually obtained by saponifying an ethylene-vinyl ester copolymer. The production and saponification of the ethylene-vinyl ester copolymer can be performed by known methods. A representative example of the vinyl ester is vinyl acetate, but other fatty acid vinyl esters such as vinyl formate, vinyl propionate, vinyl valerate, vinyl caprate, vinyl laurate, vinyl stearate, vinyl pivalate, and vinyl versatate can also be used.

The ethylene unit content of the ethylene-vinyl alcohol copolymer is preferably 20 mol% or more, and more preferably 25 mol% or more. The ethylene unit content in the ethylene-vinyl alcohol copolymer is preferably 60 mol% or less, more preferably 55 mol% or less, and even more preferably 50 mol% or less.

The saponification degree of the ethylene-vinyl alcohol copolymer is preferably 80 mol% or more, more preferably 90 mol% or more, and even more preferably 95 mol% or more. The saponification degree of the ethylene-vinyl alcohol copolymer may be 100 mol% or less, or may be 99.99 mol% or less. The saponification degree of the ethylene-vinyl alcohol copolymer can be calculated by performing $^1$H-NMR measurement and measuring the peak area of hydrogen atoms contained in the vinyl ester structure and the peak area of hydrogen atoms contained in the vinyl alcohol structure.

Further, the ethylene-vinyl alcohol copolymer may contain units derived from monomers other than ethylene, vinyl esters, and saponified products thereof, as long as the object of the present invention is not impaired. When the ethylene-vinyl alcohol copolymer contains such other monomer units, the content of such other monomer units is preferably 30 mol% or less, more preferably 20 mol% or less, even more preferably 10 mol% or less, and particularly preferably 5 mol% or less, based on the total monomer units of the ethylene-vinyl alcohol copolymer. When the ethylene-vinyl alcohol copolymer contains units derived from such other monomers, the lower limit of the content may be 0.05 mol% or may be 0.10 mol%. Examples of such other monomers include alkenes such as propylene, butylene, pentene, and hexene; alkenes having an ester group, such as 3-acyloxy-1-propene, 3-acyloxy-1-butene, 4-acyloxy-1-butene, 3,4-diacyloxy-1-butene, 3-acyloxy-4-methyl-1-butene, 4-acyloxy-2-methyl-1-butene, 4-acyloxy-3-methyl-1-butene, 3,4-diacyloxy-2-methyl-1-butene, 4-acyloxy-1-pentene, 5-acyloxy-1-pentene, 4,5-diacyloxy-1-pentene, 4-acyloxy-1-hexene, 5-acyloxy-1-hexene, 6-acyloxy-1-hexene, 5,6-diacyloxy-1-hexene, and 1,3-diacetoxy-2-methyleneprylene, or saponified products thereof; unsaturated acids such as acrylic acid, methacrylic acid, crotonic acid, and itaconic acid, or anhydrides, salts, or mono- or di-alkyl esters thereof; nitriles such as acrylonitrile and methacrylonitrile; amides such as acrylamide and methacrylamide; olefin sulfonic acids such as vinyl sulfonic acid, allyl sulfonic acid, and methallylsulfonic acid, or salts thereof; vinyl silane compounds such as vinyltrimethoxysilane, vinyltriethoxysilane, vinyltri(β-methoxy-ethoxy)silane, and γ-methacryloxypropylmethoxysilane; and alkyl vinyl ethers, vinyl ketones, N-vinylpyrrolidone, vinyl chloride, vinylidene chloride, and the like.

The ethylene-vinyl alcohol copolymer may be post-modified by urethanization, acetalization, cyanoethylation, oxyalkylenation, or the like.

The ethylene-vinyl alcohol copolymer may be used alone or in a combination of two or more.

The fiber structure (a1) may be formed of an ethylene-vinyl alcohol copolymer in combination with a polyester or the like. The fiber structure (a1) may also be composed of a composite fiber having a core-sheath structure wherein the core of the fiber is a polyester and the sheath is an ethylene vinyl alcohol copolymer. The cross-sectional structure of the composite fiber may be of a parallel type (a side-by-side type or a multilayer bonding type) or of an eccentric core-sheath type. In such a case, it is preferable that the ethylene vinyl alcohol copolymer covers 50% or more of the fiber surface. Further, the fiber structure (a1) preferably contains 50 mass% or more of the composite fiber, and may further contain fibers made of other materials. Examples of other materials include polyester.

The fibers used in the fiber structure (a1) preferably have an average fineness of 1.0 to 10 dtex. A fineness within the above range is preferable because it is easy to obtain spaces for capturing cells or a cell cluster.

The average fiber length of the fibers used in the fiber structure (a1) is not particularly limited, and the fibers can be long fibers or short fibers. When the fiber structure (a1) is produced by the CAD method, the average fiber length is preferably 32 to 62 mm from the viewpoint of spinnability.

The fiber structure (a1) may be composed of one fiber structure or a laminate of two or more fiber structures.

The fiber structure (a1) preferably has a porosity of 90% or more, more preferably 93% or more, and even more preferably 95% or more. A porosity between the fibers in the fiber structure (a1) within the above range is preferable because it allows cells or a cell cluster to be easily captured in the fiber structure (a1). The upper limit of the porosity of the fiber structure (a1) is not particularly limited, but is preferably, for example, 99.9% or less, more preferably 99.5% or less, and even more preferably 99.0% or less. For example, the porosity of the fiber structure (a1) is preferably 90.0 to 99.9%, more preferably from 93.0 to 99.5%, and even more preferably from 95.0 to 99.0%.

In the present specification, the porosity can be calculated from the basis weight C (g/m$^2$), thickness D (cm), and average specific gravity E (g/cm$^2$), of the fiber structure according to the following formula.

$$\text{Porosity (\%)} = 100 - ((C/D/E) \times 10^{-4} \times 100)$$

The thickness of the fiber structure (a1) is preferably 100 to 2000 μm, more preferably 300 to 1500 μm, and even more preferably 300 to 1000 μm. A thickness within the above range is preferable because a sufficient number of cells can be retained and the supply of oxygen etc. to the cells is unlikely to be hindered.

The basis weight of the fiber structure (a1) is preferably 20 to 300 g/m$^2$, more preferably 30 to 200 g/m$^2$, and even

more preferably 40 to 100 g/m$^2$.

The area of the fiber structure (a1) is not particularly limited, but is preferably 1.5 to 150 cm$^2$.

The method for producing the fiber structure (a1) is not particularly limited. However, it is preferable to form bonding points between the fibers by thermal bonding, and the steam jet method is preferably used to provide a space in which cells or a cell cluster can be captured and to obtain hardness against compression.

Dense Fiber Structure (a2)

The cell-capturing layer (A) preferably comprises, in addition to the fiber structure (a1), a dense fiber structure (a2) that has the function of preventing the escape of cells or a cell cluster captured in the fiber structure (a1). The dense fiber structure (a2) may be laminated on one side of the fiber structure (a1) in the order of dense fiber structure (a2)/fiber structure (a1) or the order of fiber structure (a1)/dense fiber structure (a2), or may be laminated on both sides of the fiber structure (a1) to form a multilayer of the dense fiber structure (a2)/fiber structure (a1)/dense fiber structure (a2). The dense fiber structure (a2) may be disposed around the fiber structure (a1) so as to cover a part or the whole of the fiber structure (a1) with the dense fiber structure (a2). In order to prevent the escape of cells or a cell cluster captured in the fiber structure (a1), either forming a laminate of the dense fiber structure (a2)/fiber structure (a1)/dense fiber structure (a2) or disposing the dense fiber structure (a2) around the fiber structure (a1) to cover the entire fiber structure (a1) is preferable. It is more preferable that the dense fiber structure (a2) is disposed so as to cover the entire periphery of the fiber structure (a1). When lamination of the dense fiber structure (a2)/fiber structure (a1)/dense fiber structure (a2) is done, the dense fiber structures (a2) are preferably laminated on the fiber structure (a1) in such a manner as to cover the entire periphery of the fiber structure (a1).

The function of preventing the escape of cells or a cell cluster means that cells or a cell cluster are captured without escaping from the cell-capturing layer. For example, it means that the cell viability evaluated in the Examples below is 80% or more.

The dense fiber structure (a2) may be a nonwoven fabric, a woven fabric, or a knitted fabric, with a nonwoven fabric being preferred because the pore size can be easily controlled by the production conditions. The material of the dense fiber structure (a2) preferably contains at least one member selected from the group consisting of ethylene-vinyl alcohol copolymers and cellulose acetate, and more preferably contains an ethylene-vinyl alcohol copolymer. Examples of ethylene-vinyl alcohol copolymers that can be preferably used are those described in the explanation of the fiber structure (a1). The dense fiber structure (a2) may also be composed of a composite fiber having a core-sheath structure wherein the core of the fiber is a polyester and the sheath is an ethylene vinylalcohol copolymer.

The dense fiber structure (a2) preferably has an average pore size of 5 μm or more and 35 μm or less, and more preferably 5 μm or more and 25 μm or less. When the average pore size of the dense fiber structure (a2) is 5 μm or more, the supply of oxygen and the like to the cells or a cell cluster captured in the fiber structure (a1) is less likely to be hindered. The average pore size of the dense fiber structure (a2) of 35 μm or less is preferable because the cells or a cell cluster captured in the fiber structure (a1) are less likely to escape from the fiber structure (a1).

In the present specification, the average pore size can be measured by the following procedure. Photographs of the surface of the fiber structure (a2) are taken at a magnification of 10x under irradiation of transmitted light using a stereomicroscope. Using image analysis software, 50 or more bright spots through which light transmits are extracted from the microphotograph, and the area of each bright spot is measured. The diameter calculated as the area of a perfect circle is taken as the pore size of the bright spot. The average pore size is determined by calculating the average pore size of the 50 or more bright spots.

The dense fiber structure (a2) preferably has a thickness of 1500 μm or less, more preferably 1000 μm or less, even more preferably 500 μm or less, and particularly preferably 300 μm or less. The thickness of the dense fiber structure (a2) is preferably 50 μm or more, and more preferably 100 μm or more. A thickness of 50 to 1500 μm is preferable because the supply of oxygen and the like to the cells is less likely to be hindered.

The dense fiber structure (a2) preferably has a basis weight of 3 to 300 g/m$^2$, and more preferably 10 to 100 g/m$^2$. A basis weight within the above range is preferable because the preferred average pore size and the preferred thickness can be achieved.

The dense fiber structure (a2) preferably has a porosity of less than 90%, more preferably 87% or less, even more preferably 85% or less, and particularly preferably 80% or less. The porosity of the dense fiber structure (a2) is preferably 30% or more, more preferably 50% or more, even more preferably 60% or more, and particularly preferably 70% or more. A porosity between the fibers in the dense fiber structure (a2) within the above range is preferable because it tends to enhance the function of preventing the escape of cells or a cell cluster. For example, the porosity of the dense fiber structure (a2) is preferably 30.0 to 89.9%, more preferably 50.0 to 87.0%, even more preferably 60.0 to 85.0%, and particularly preferably 70.0 to 80.0%.

The area of the dense fiber structure (a2) is not particularly limited, but is preferably 1.0 to 400 cm$^2$, more preferably 1.0 to 200 cm$^2$, and even more preferably 1.5 to 150 cm$^2$. The area of the dense fiber structure (a2) is preferably larger than that of the fiber structure (a1) so that the dense fiber structure (a2) can cover the fiber structure (a1).

The method for producing the dense fiber structure (a2) is not particularly limited, but the melt blowing method is preferably used in order to achieve a suitable average pore size. The surface of the dense fiber structure (a2) may be subjected to calendaring.

The number of cells retained in the cell-capturing layer (A) is not particularly limited, but is preferably $1 \times 10^5$ to $1 \times 10^7$ cells/cm$^3$, and more preferably $1 \times 10^6$ to $1 \times 10^7$ cells/cm$^3$.

Immunoisolation Layer (B)

The immunoisolation device of the present invention comprises an immunoisolation layer (B) that covers the cell-capturing layer (A). In a typical embodiment, from the viewpoint of obtaining a sufficient immunoisolation effect, it is preferable that the immunoisolation layer (B) in the device of the present invention covers the entire surface of the cell-capturing layer (A). For the same reason, the immunoisolation layer preferably does not have any pores such as pinholes penetrating the membrane.

The immunoisolation layer (B) is preferably an immunoisolation multilayer (B') comprising a porous membrane (b1) or fiber structure (b2) and a hydrogel (b3).

Porous Membrane (b1)

The porous membrane (b1) of the immunoisolation layer (B) is a membrane having multiple pores. Being a porous membrane can be confirmed by scanning electron microscope (SEM) or transmission electron microscope (TEM) images of the cross-section of the membrane. The porous membrane is preferably a semi-permeable membrane.

The thickness of the porous membrane (b1) is not particularly limited, but is preferably 300 μm or less, more preferably 15 μm to 290 μm, and even more preferably 30 μm to 150 μm. A thickness within the above range is preferable because the supply of oxygen and other substances to the cells is less likely to be hindered while the strength of the immunoisolation layer (B) is maintained.

The average pore size of the porous membrane (b1) is not particularly limited, but is preferably 0.01 μm to 10 μm, more preferably 0.01 μm to 5 μm, and even more preferably 0.01 to 3 μm. The average pore size can be determined from SEM images or TEM images. For example, the surface of the porous membrane is observed using SEM, and 50 pores are randomly selected from pores formed on the surface. The major diameter of each pore is measured, and the average of the major diameters of 50 pores is calculated to obtain the average pore size.

The maximum pore size of the porous membrane (b1) is not particularly limited, but is preferably 0.01 μm to 10 μm, more preferably 0.01 μm to 5 μm, and even more preferably 0.01 μm to 4 μm. When the maximum pore size is within the above range, the invasion of immune response cells and immune system humoral factors into the device can be inhibited, while nutrients such as amino acids, vitamins, inorganic salts, glucose, and like carbon sources, and physiologically active substances such as oxygen, carbon dioxide, cytokines, hormones, and insulin, are allowed to permeate sufficiently. The maximum pore size can be determined from SEM images or TEM images. For example, the surface of the porous membrane is observed using SEM, and 50 pores are randomly selected from pores formed on the surface. The major diameter of each pore is measured, and the maximum value among the 50 major diameters is defined as the maximum pore size.

Since it is necessary to have the functions of inhibiting cell infiltration from the recipient and also prevent leakage of cells, which are a material to be transplanted, the average pore size or the maximum pore size of the porous membrane (b1) is desirably smaller than the cell size and is 5 μm or less.

The porous membrane (b1) comprises a polymer. Preferably, the porous membrane (b1) is substantially composed of a polymer.

Examples of the polymer include thermoplastic resins and thermosetting resins. Specific examples of the polymer include ethylene-vinyl alcohol copolymers, polysulfones, cellulose acetates such as cellulose acetate, nitrocellulose, sulfonated polysulfones, polyethersulfones, polyacrylonitrile, styrene-acrylonitrile copolymers, styrene-butadiene copolymers, polyvinyl alcohols, polycarbonates, organosiloxane-polycarbonate copolymers, polyester carbonates, organopolysiloxanes, polyphenylene oxides, polyamides, polyimides, polyamideimides, polybenzimidazoles, and polytetrafluoroethylene (PTFE). In view of solubility, optical properties, electrical properties, strength, elasticity, etc., the polymer may be a homopolymer or may be a copolymer, a polymer blend, a polymer alloy, or the like. The polymer that constitutes the porous membrane may contain a hydrophilic polymer such as polyvinylpyrrolidone, hydroxypropyl cellulose, hydroxyethyl cellulose, or polyethylene glycol. Combining a hydrophilic polymer and a hydrophobic polymer can improve biocompatibility.

The polymer for forming the porous membrane (b1) is preferably a highly biocompatible material that is unlikely to cause adhesion to the recipient's tissue around the transplantation site or incur inflammation, etc. The porous membrane (b1) preferably contains at least one member selected from the group consisting of ethylene-vinyl alcohol copolymers and cellulose acetate, and more preferably contains an ethylene-vinyl alcohol copolymer. Examples of ethylene-vinyl alcohol copolymers that can be preferably used are those described in the explanation of the fiber structure (a1).

The porous membrane (b1) may be composed of one kind of porous membrane or may be formed of laminate of two or more kinds of porous membranes. When the porous membrane (b1) comprises two or more porous membranes, the porous membranes may be directly laminated, or a hydrogel or a fiber structure may be interposed between the two porous membranes.

In a preferred embodiment, the porous membrane is a single layer formed of one composition. In a preferred embodiment, the porous membrane does not have a lamination structure comprising multiple layers.

Fiber Structure (b2)

The fiber structure (b2) of the immunoisolation layer (B) is, for example, a nonwoven fabric, a woven fabric, or a knitted fabric. The fiber structure (b2) is preferably a nonwoven fabric. The fiber structure is formed by bonding or entangling fibers together by heat, mechanical, or chemical action. The basis weight (weight per unit area) can be adjusted by adjusting the fiber diameter and/or fiber amount, so that in addition to the strength, the permeability, filtration, etc. can be controlled. The fiber structure (b2) preferably has a basis weight of 10 to 100 g/m$^2$. Considering the diffusion efficiency of physiologically active substances from the material to be transplanted, the thickness of the fiber structure (b2) is preferably 300 $\mu$m or less, and is more preferably is 200 $\mu$m or less and as thin as possible. The lower limit of the thickness of the fiber structure (b2) is not particularly limited, but is, for example, preferably 50 $\mu$m or more, and more preferably 100 $\mu$m or more.

Examples of fiber materials of the fiber structure (b2) include gelatin, collagen, chitin, chitosan, fibronectin, dextran, cellulose, polyethylene (PE), polypropylene (PP), polyurethane, polyamide, polyester, polyvinyl alcohol (PVA), ethylene-vinyl alcohol copolymers, polylactic acid, polyglycolic acid, polylactic acid-polyglycolic acid copolymers, PVAs modified with a monomer such as methacrylic-modified PVA and acrylic-modified PVA, polycaprolactone, polyglycerol sebacic acid, polyhydroxyalkanoic acid, polybutylene succinate, polymethylene carbonate, cellulose diacetate, cellulose triacetate, methylcellulose, propylcellulose, benzyl cellulose, cellulose acetates such as carboxymethyl cellulose, fibroin, and silk. The fiber material of the fiber structure, as well as the porous membrane (b1), is preferably biocompatible. The fiber structure (b2) preferably contains at least one member selected from the group consisting of ethylene-vinyl alcohol copolymers and cellulose acetate, and more preferably contains an ethylene-vinyl alcohol copolymer. Examples of ethylene-vinyl alcohol copolymers that can be preferably used include those described in the explanation of the fiber structure (a1). The surface of the fiber structure is preferably smoothed by heat, mechanical, or chemical treatment.

Hydrogel (b3)

Examples of hydrosols for producing the hydrogel (b3) of the immunoisolation layer (B) include sols that gel in the presence of a metal ion to form hydrogels; sols that gel in response to pH to form hydrogels; and sols that gel in response to light to form hydrogels. Metal ions and pH are examples of the chemical action. In order to gel such hydrosols, an operation such as bringing a metal ion into contact with a hydrosol, adjusting the temperature to gelling conditions, adjusting the pH to gelling conditions, applying light under gelling conditions, or providing a magnetic field under gelling conditions may be performed depending on the characteristics of the gel used.

Examples of hydrogels obtainable by gelling in the presence of a metal ion include alginate gels obtainable by gelling in the presence of a divalent or trivalent metal ion, preferably an alkaline earth metal ion, such as a calcium ion or a magnesium ion; carrageenan gels obtainable by gelling in the presence of a calcium ion and/or a potassium ion; and acrylic acid-based synthesis gels obtainable by gelling in the presence of a sodium ion.

Examples of pH-responsive hydrogels include alginate gels, chitosan gels, carboxymethyl cellulose gels, and acrylic acid-based synthetic gel.

Examples of photoresponsive hydrogels include a synthesis gel in which azobenzene and cyclodextrin are combined in the skeleton, a gel composed of a supramolecule having fumaric acid amide as a spacer, a gel obtainable by crosslinking or bonding via a nitrobenzyl group, and a gel composed of modified polyvinyl alcohol.

The modified polyvinyl alcohol may be, for example, a (meth)acryloyl group-modified polyvinyl alcohol. The (meth)acryloyl group can be introduced by subjecting a hydroxyl group on the side chain of polyvinyl alcohol to an esterification reaction or an ester exchange reaction with an ethylenically unsaturated group-containing compound in the presence of a base. Examples of the ethylenically unsaturated group-containing compound include (meth)acrylic acid or derivatives thereof, such as (meth)acrylic acid, (meth)acrylic anhydride, (meth)acrylic acid halide, and (meth)acrylic acid ester.

Preferred examples of the hydrogel (b3) include polyvinyl alcohol, polyethylene glycol, chitosan, alginate, and the like. The hydrogel (b3) preferably contains at least one member selected from the group consisting of polyvinyl alcohols and polyethylene glycols, and more preferably contains a polyvinyl alcohol. The polyvinyl alcohol-based polymer can be produced, for example, by saponification of a polyvinyl ester obtainable by polymerizing a vinyl ester-based monomer, and converting the ester group in the polyvinyl ester to a hydroxyl group.

Examples of the vinyl ester-based monomer include aliphatic vinyl esters such as vinyl formate, vinyl acetate, vinyl propionate, vinyl n-butyrate, vinyl isobutyrate, vinyl pivalate, vinyl versatate, vinyl caproate, vinyl caprylate, vinyl caprate, vinyl laurate, vinyl myristate, vinyl palmitate, vinyl stearate, and vinyl oleate; and aromatic vinyl ester such as vinyl benzoate. These vinyl ester-based monomers may be used alone or in a combination of two or more.

Among the vinyl ester-based monomers, aliphatic vinyl esters are preferred, and vinyl acetate is more preferred in view of production cost. In other words, the polyvinyl ester is preferably polyvinyl acetate obtainable by polymerization of vinyl acetate.

The polyvinyl ester may optionally contain structural units derived from monomers other than vinyl ester monomers as long as the effect of the present invention is not impaired. Examples of such other monomers include α-olefins such as ethylene, propylene, n-butene, and isobutylene; acrylic acid or salts thereof; alkyl acrylate compounds such as methyl acrylate, ethyl acrylate, n-propyl acrylate, i-propyl acrylate, n-butyl acrylate, i-butyl acrylate, t-butyl acrylate, 2-ethylhexyl acrylate, dodecyl acrylate, and octadecyl acrylate; methacrylic acid or salts thereof; alkyl methacrylate compounds such as methyl methacrylate, ethyl methacrylate, n-propyl methacrylate, i-propyl methacate, n-butyl methacrylate, i-butyl methacrylate, t-butyl methacrylate, 2-ethylhexyl methacrylate, dodecyl methacrylate, and octadecyl methacrylate; acrylamide derivatives such as acrylamide, N-methylacrylamide, N-ethylacrylamide, N,N-dimethylacrylamide, diacetoneacrylamide, acrylamidopropane sulfonic acid or salts thereof, acrylamidopropyl dimethylamine or salts or quaternary salts thereof, and N-methylolacrylamide or derivatives thereof; methacrylamide derivatives such as methacrylamide, N-methylmethacrylamide, N-ethylmethacrylamide, methacrylamidopropanesulfonic acid or salts thereof, methacrylamidopropyl dimethylamine or salts or quaternary salts thereof, N-methylolmethacrylamide or derivatives thereof; N-vinylamide derivatives such as N-vinylformamide and N-vinylacetamide; vinyl ether compounds such as methyl vinyl ether, ethyl vinyl ether, n-propyl vinyl ether, i-propyl vinyl ether, n-butyl vinyl ether, i-butyl vinyl ether, t-butyl vinyl ether, dodecyl vinyl ether, and stearyl vinyl ether; nitrile compounds such as acrylonitrile and methacrylonitrile; vinyl halides such as vinyl chloride and vinyl fluoride; vinylidene halides such as vinylidene chloride and vinylidene fluoride; allyl compounds such as allyl acetate and allyl chloride; maleic acid or salts, esters, or acid anhydrides thereof; vinyl silyl compounds such as vinyl trimethoxysilane; and isopropenyl acetate. These can be used alone or in a combination of two or more.

The average polymerization degree of the polyvinyl alcohol is preferably 300 to 10000, more preferably 500 to 5000, even more preferably 1000 to 5000, still even more preferably 1500 to 5000, and particularly preferably 2000 to 5000. A thickness within the above range is preferred in view of permeability of substances and ease of handling in a multilayer formation.

The average polymerization degree of the polyvinyl alcohol in the present specification refers to the average polymerization degree measured according to JIS K 6726:1994. Specifically, the average polymerization degree can be determined from the intrinsic viscosity that is measured in water at 30°C after the raw material PVA has been saponified and purified.

From the viewpoint of improving the water solubility of the polyvinyl alcohol, the degree of saponification of the polyvinyl alcohol is preferably 50 mol% or more, more preferably 60 mol% or more, and even more preferably 65 mol% or more.

From the viewpoint of inhibiting the hydrosol solution from having high viscosity and improving the storage stability of the hydrosol solution, the degree of saponification of the polyvinyl alcohol is preferably 99.9 mol% or less, more preferably 99.5 mol% or less, and even more preferably 99.0 mol % or less.

In the present specification, the degree of saponification of polyvinyl alcohol means the ratio (mol%) of the number of moles of the vinyl alcohol unit to the total number of moles of the structural unit (e.g., vinyl acetate unit) that can be converted to a vinyl alcohol unit by saponification in the raw material PVA and the vinyl alcohol unit. The degree of saponification can be measured according to JIS K 6726:1994.

The thickness of the hydrogel (b3) is not particularly limited, but is preferably 1 to 300 μm, more preferably 5 to 200 μm, and even more preferably 10 to 100 μm.

The crosslinking of the hydrogel (b3) makes it possible to adjust the permeability, strength, etc. of nutrients such as glucose, physiologically active substances such as insulin, and immune system humoral factors.

The gel strength of the hydrogel (b3) is preferably 20 to 300 kPa, and more preferably 50 to 200 kPa. The gel strength can be measured by the following procedure using a tensile tester.

First, the hydrosol solution is poured between two glass plates that are separated from each other by inserting 1 mm-thick spacers therebetween and treated under the predetermined gelling conditions to obtain a gel sheet with a thickness of 1 mm. A test piece is cut out from the gel sheet using a dumbbell cutter specified in JIS K 6251-3. The test piece is set on a tensile tester (model 5566) produced by Instron Co., Ltd., and the breaking stress and breaking strain were measured while acquiring image data. The stress at which the test piece breaks is defined as the gel strength.

Configuration of Immunoisolation Layer (B) etc.

The immunoisolation layer (B) is preferably a porous membrane (b1) comprising at least one member selected from the group consisting of ethylene-vinyl alcohol copolymers and cellulose acetate, or a fiber structure (b2) comprising at least one member selected from the group consisting of ethylene-vinyl alcohol copolymers and cellulose acetate. Further, the immunoisolation layer (B) may have a multilayer structure containing, in addition to the porous membrane (b1) or the fiber structure (b2), one or more layers other than the porous membrane (b1) and the fiber structure (b2). A preferable examples of layers other than the porous membrane (b1) and the fiber structure (b2) is a hydrogel (b3). In the present specification, an immunoisolation layer comprising a porous membrane (b1) or a fiber structure (b2), and a hydrogel (b3) may be referred to as an immunoisolation multilayer (B').

Forming a multilayer with a hydrogel (b3) not only improves immunoisolation but also increases the strength of the immunoisolation layer (B). Achieving both permeability and immunoisolation can be adjusted by adjusting the pore size of the porous membrane and the gel strength and/or degree of crosslinking of the hydrogel (b3).

Cell infiltration and cell leakage can be inhibited by the porous membrane (b1). However, it is not easy to suppress the infiltration of immune system humoral factors, such as IgG antibodies, while the permeability of necessary physiologically active substances is not reduced. Accordingly, by adjusting the gel strength or crosslinking density of the hydrogel (b3) that forms a multilayer with the porous membrane (b1), the infiltration of immune system humoral factors such as IgG antibodies can be inhibited without reducing the permeability of physiologically active substances.

When the fiber structure layer (b2) used is a single layer, it is not easy to inhibit the infiltration of immune system humoral factors, such as IgG antibodies, as well as cell infiltration and cell leakage, while the permeability of necessary physiologically active substances is not reduced. Accordingly, by adjusting the gel strength or crosslinking density of the hydrogel (b3) that forms a multilayer with the porous membrane (b2), the infiltration of immune system humoral factors such as IgG antibodies, as well as cell infiltration and cell leakage, can be inhibited without reducing the permeability of physiologically active substances.

The porous membrane (b1), the fiber structure (b2), and the hydrogel (b3) each form a layer, and the layers may be clearly separated at their boundaries, or the boundary between two adjacent layers may not be clearly defined, or two or three different kinds of layers may be integrated to form one layer.

For example, when the immunoisolation layer comprises two kinds of layers that are a fiber structure (b2) and a hydrogel (b3), the immunoisolation layer may have a structure in which the fiber structure (b2) and the hydrogel (b3) are clearly separated, or a mixed layer of the fiber structure and the hydrogel may be present between the fiber structure and the hydrogel, or the fiber structure and the hydrogel may be completely integrated into one layer.

When the immunoisolation layer comprises two kinds of layers that are a fiber structure (b2) and a porous membrane (b1), the immunoisolation layer may have a structure in which the fiber structure and the porous membrane are clearly separated, or a mixed layer of the fiber structure and the porous membrane may be present between the fiber structure and the hydrogel, or the fiber structure and the porous membrane may be completely integrated into one layer.

When the immunoisolation layer comprises two kinds of layers that are a hydrogel (b3) and a porous membrane (b1), the immunoisolation layer may have a structure in which the hydrogel and the porous membrane are clearly separated, or a mixed layer of the hydrogel and the porous membrane may be present between the hydrogel and the porous membrane, or the hydrogel and the porous membrane may be completely integrated into one layer.

The immunoisolation device according to a particularly preferred embodiment of the present invention is composed of any one of the following three multilayer structures (i) to (iii):

(i) A porous membrane (b1) is used as a substrate, and a hydrogel (b3) is applied to the porous membrane (b1) or the porous membrane (b1) is impregnated with a hydrogel (b3) to form a multilayer structure.
(ii) A fiber structure (b2) used as a substrate, and a hydrogel (b3) is applied to the fiber structure (b2) or the fiber structure (b2) is impregnated with a hydrogel (b3) to form a multilayer structure.
(iii) A structural fiber (b2) is used as a substrate, and a porous membrane (b1) is formed on the structural fiber (b2). Further, a hydrogel (b3) is applied to the porous membrane (b1) or the porous membrane (b1) is impregnated with a hydrogel (b3) to form a multilayer structure.

When the immunoisolation layer (B) comprises multiple layers, the layers may be bonded by an adhesive, heat, pressure, or the like. When adjacent layers are formed of highly compatible materials, the layers can be bonded by sequentially forming the layers.

Specifically, a porous membrane (b1) is used as a substrate, and a hydrosol solution is directly applied to the porous membrane and formed into a hydrogel by heat, temperature, light, or chemical action to form a multilayer structure. Alternatively, a fiber structure (b2) is used as a substrate, and a hydrosol solution is directly applied to the fiber structure and formed into a hydrogel by heat, temperature, light, or chemical action to form a multilayer structure. When a fiber structure (b2) such as a nonwoven fabric coated with a hydrogel (b3) is used instead of the porous membrane (b1), an immunoisolation layer (B) that has higher substance permeability and higher strength than the porous membrane (b1) can be formed while the hydrogel (b3) ensures immunoisolation.

The hydrosol solution preferably has a solids concentration of 3 to 15 mass%, more preferably 3 to 10 mass%, even more preferably 3 to 8 mass%, and particularly preferably 3 to 5 mass%. A solids concentration within the above range is preferable because the permeability of immune system liquid factors such as IgG can be inhibited while the permeability of substances such as glucose and insulin is maintained.

Alternatively, a hydrogel (b1) is used as a substrate, and a polymer solution, which is a raw material for a porous membrane (b1), is directly applied to the substrate. The raw material for a porous membrane (b1) is solidified by phase separation, which is a phase transition phenomenon, to thereby form a porous membrane (b1). Some raw materials for the porous membrane (b1) may require pre-treatment to reduce the water content by pre-drying the hydrogel (b3).

In one preferred embodiment of the present invention, a fiber structure (b2) with excellent durability is used as a substrate; and a hydrogel (b3) alone or both a porous membrane (b1) and a hydrogel (b3) are formed on the fiber structure (b2) to thereby achieve both a reduction in thickness of the device, which improves the diffusion efficiency of physiologically active substances, and durability due to increased strength, while maintaining the immunoisolation effect.

The thickness of the immunoisolation layer (B) is not particularly limited, but is preferably 10 $\mu$m or more and 500 $\mu$m or less, more preferably 300 $\mu$m or less, even more preferably 200 $\mu$m or less, still even more preferably 170 $\mu$m or less, and particularly preferably 150 $\mu$m or less. Even when the immunoisolation layer (B) is an immunoisolation multilayer (B'), preferable thickness of the immunoisolation layer is also within the above range. Considering the mass diffusion efficiency of the physiologically active substance from the material to be transplanted, the thickness of the immunoisolation multilayer (B) is preferably 500 $\mu$m or less, more preferably 300 $\mu$m or less, and even more preferably 100 $\mu$m or less and is as thin as possible.

The area of the immunoisolation layer (B) is not particularly limited, but is preferably 1.0 to 400 $cm^2$, more preferably 1.0 to 200 $cm^2$, and even more preferably 1.5 to 150 $cm^2$. The area of the immunoisolation layer (B) is preferably equal to or greater than that of the cell-capturing layer (A) so as to cover the cell-capturing layer (A).

The outermost layer of the immunoisolation layer (B) is preferably biocompatible in order to prevent the immunoisolation layer from being recognized as foreign matter. The immunoisolation layer is required to have permeability necessary to allow for sufficient permeation of oxygen and nutrients into the materials to be transplanted, which is present inside of the device.

The outermost layer of the immunoisolation layer (B) may be any of a porous membrane (b1), a fiber structure (b2), or a hydrogel (b3), or a mixture of two or three of these. The innermost layer of the immunoisolation membrane (B) may be any of a porous membrane (b1), a fiber structure (b2), or a hydrogel (b3), or a mixture of two or three of these. In the present specification, the outermost layer of the immunoisolation layer (B) means the layer that constitutes the outer part of the immunoisolation device of the present invention in the immunoisolation layer (B), i.e., the layer that contacts with the tissue surrounding the transplantation site (host), and the innermost layer of the immunoisolation layer (B) means the layer that constitutes the part of the immunoisolation layer (B) that contacts the cell-capturing layer (A) in the immunoisolation device of the present invention (the inner portion).

When the outermost layer of the immunoisolation layer (B) is a porous membrane (b1), the porous membrane (b1) is desirably made of a material that is more biocompatible than hydrogel (b3), and also serves to prevent adhesion of the hydrogel (b3) to the recipient's tissue and the induction of inflammation etc.

When the outermost layer of the immunoisolation layer (B) is a fiber structure (b2), the fiber structure (b2) is desirably a material with higher biocompatibility than the hydrogel (b3). Since the outermost layer also serves to protect the hydrogel from adhering to the recipient's tissue at the transplantation site and inducing inflammation, etc., the surface of the fiber structure (b2) as the outermost layer is desirably smoothed by heat, mechanical, or chemical treatment.

By modifying the hydrogel (b3) to inhibit the induction of inflammatory reactions, it is also possible to use the hydrogel (b3) as the outermost layer and use the porous membrane (b1) as the innermost layer. In that case, it is possible to load a physiologically active substance on the hydrogel and impart functionality such as induction of angiogenesis.

Material Permeability of Immunoisolation Layer (B)

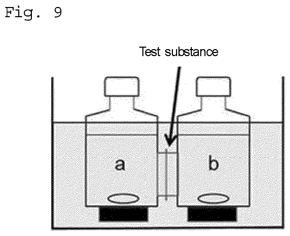

The permeation amount of glucose, insulin, an immune system humoral factor through the immunoisolation layer (B) can be measured by inserting the immunoisolation layer (B) at the connection site between two glass chambers having the same volume, pouring a sample solution of a known concentration of insulin or the like into chamber a, pouring water into chamber b, and quantifying, with stirring at 37°C, the amount of insulin or the like contained in the liquid sampled from chamber b after a certain period of time by ELISA or the like (Fig. 9). Note that the amounts of liquids in chambers a and b are adjusted to be equal when the sample solution is poured into chamber a.

The permeability of glucose, insulin, immune system humoral factors, etc. through the immunoisolation layer is expressed as a percentage that represents what the amount of each substance having permeated into chamber b, as measured by the above method after 20 hours, is relative to the concentration at which the equilibrium is reached, i.e., half the concentration of the substance poured into chamber a. More specifically, for example, the permeability can be obtained according to the following formula:

**Permeability (%) of each substance = Concentration of each substance in chamber b after --> 20 hours of measurement / (Concentration of each substance in chamber a at the start of measurement ÷ 2) × 100**

The insulin permeability and glucose permeability of the immunoisolation layer (B) of the present invention are preferably 50% or more, more preferably 90% or more, and even more preferably 95% or more.

The immune system humoral factor permeability (B) of the immunoisolation layer of the present invention is preferably 30% or less, and more preferably 10% or less.

The permeability of each material can be controlled by the pore size of the porous membrane (b1), the fiber basis weight of the fiber structure (b2), or the gel strength and degree of crosslinking of the hydrogel (b3). In order to exhibit the immune isolation function, it is desirable that the pore size of the porous membrane (b1) is equal to or smaller than a size that does not allow permeation of cells. It is desirable that the hydrogel (b3) does not inhibit the permeation of physiologically active substances but can inhibit the permeation of immune response factors such as cells and antibodies.

Examples of immunoresponsive cells include macrophages, cytotoxic T cells, natural killer cells, dendritic cells, and helper T cells. Examples of immune system humoral factors include antibodies, complements, and cytokines.

Immunoisolation Device

The immunoisolation device according to a preferred embodiment of the present invention is in the form of a pouch, a tube, a cylinder, a rectangular tube, a sphere, a cube, a rectangular solid, a sheet, or hollow fibers, and a cell-capturing layer (A) is enclosed in the device. The material to be transplanted is introduced into the cell-capturing layer (A). Examples of the material to be transplanted include cells, cell clusters, cell sheets, grafts, and the like. Other examples of the material to be transplanted include physiologically active substances other than cells, such as enzymes, hormones, cytokines, and drugs. Preferred cells, cell clusters, or implants are those that release physiologically active substances from the immunoisolation device. That is, in a preferred embodiment, cells, cell clusters, and grafts include physiologically active substance-producing cells.

To introduce the material to be transplanted into the cell-capturing layer (A), a suspension of the material to be transplanted is uniformly injected and seeded into the fiber structure (a1) with a pipette or the like. After seeding, a dense fiber structure (a2) of a different specification having smaller pores is disposed around the fiber structure (a1) to cover the fiber structure (a1) with the dense fiber structure (a2), thereby preventing the material to be transplanted from escape from the cell-capturing layer. That is, the porosity of the dense fiber structure (a2) is preferably smaller than the porosity of the fiber structure (a1).

The immunoisolation device can be produced, for example, in the following manner. After an immunoisolation layer (B) is disposed around the cell-capturing layer (A) prepared by the above method to cover the cell-capturing layer (A) with the immunoisolation layer (B), the peripheral portion is sealed by heat fusion to mold the device into a pouch shape. Alternatively, after an opening is provided in the device prepared in advance using an immunoisolation layer (B) and a cell-capturing layer (A) is inserted from the opening, the opening is then closed to thereby block the invasion of immunoresponsive cells and immune system humoral factors from the opening. Since oxygen, nutrients, etc. can permeate through the immunoisolation layer in portions other than the opening, the opening can be closed to inhibit the permeation of substances, including nutrients.

In heat fusion, a resin can be interposed between the immunoisolation layers (B) (immunoisolation multilayers (B')) and then heat fusion can be performed. By performing heat fusion in the state that a resin is interposed, heat fusion can be easily performed even when the innermost layers of the upper and lower immunoisolation layers (B) in the immunoisolation device are both a hydrogel (when the hydrogels of the upper and lower immunoisolation layers (B) are facing each other). The resin used in heat fusion is not particularly limited, but is preferably at least one member selected from the group consisting of ethylene-vinyl alcohol copolymers and cellulose acetate, and more preferably an ethylene-vinyl alcohol copolymer. Alternatively, heat fusion can be performed with a dense fiber structure (a2) being interposed between the immunoisolation layers (B).

After the immunoisolation device of the present invention is implanted into a living body, the material transplanted whose function has been reduced may be removed, and a new functional material to be transplanted may be introduced. This operation can be repeated. The immunoisolation device can be repeatedly used to introduce a material to be transplanted, or the immunoisolation device may be removed together with the material transplanted.

Since the immunoisolation device of the present invention has sufficient strength, can be stably present in the recipient's body, and can inhibit the invasion of immunocompetent cells into the cell-capturing layer (A), invasion of the material tod be transplanted, which is present inside of the cell-capturing layer (A), into the recipient's body, can also be

EP 4 516 329 A1

inhibited simultaneously. Accordingly, even iPS-derived cells, for which the risk of canceration of the material to be transplanted is a concern, can be used safely.

In a preferred embodiment, the immunoisolation device of the present invention preferably has shape retention in order to ensure sufficient strength in vivo.

In order to maintain functionality as an immunoisolation device, it is desirable that the fiber structure (a1) and dense fiber structure (a2) used as the cell-capturing layer (A), and the porous membrane (b1), fiber structure (b2), and hydrogel (b3) used as the immunoisolation layer (B) are all made of materials that are safe and highly biocompatible.

In order to prevent adhesion to the surrounding tissue and prevent fibrosis, the immunoisolation layer (B) is desirably composed of a material with excellent biocompatibility. When the immunoisolation layer (B) is composed of multiple materials, it is desirable that a material with excellent biocompatibility is disposed at the transplantation side, i.e., on the contact surface of the outermost layer that comes into contact with the transplantation site in a recipient. Examples of the material having excellent biocompatibility are ethylene-vinyl alcohol copolymers.

The thickness of the immunoisolation device of the present invention varies depending on the recipient's tissue, the material to be transplanted, etc., and is not particularly limited, but is preferably 400 to 2000 $\mu$m. The area of the immunoisolation device of the present invention also varies depending on the recipient's tissue, the material to be transplanted, etc., and is not particularly limited, but is preferably 1.0 to 200 cm$^2$.

Preferred embodiments of the present invention are described below with reference to the drawings.

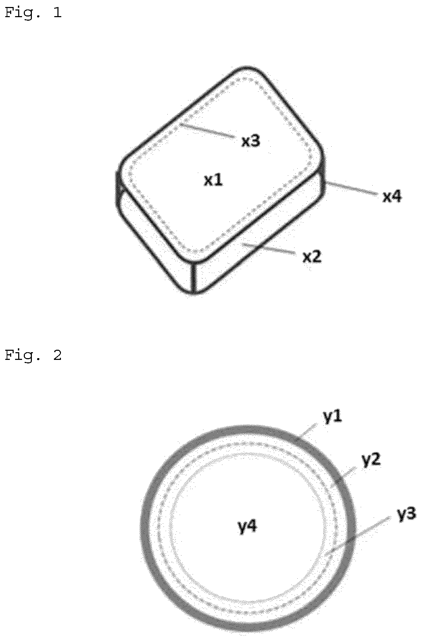

Fig. 1 is a schematic view of a device produced by molding the immunoisolation layer (B) into a pouch-like shape. Fig. 2 is a schematic view of a device produced by molding an immunoisolation layer into a tubular shape. The pouch-like device (Fig. 1) is molded by applying heat, ultrasound, high frequency, or electron beam to fuse two sheets (x1 and x2) of the immunoisolation multilayer (B) shown below (x3) with a certain distance (x4) therebetween to ensure a space for enclosing a cell-capturing layer (A) therein. Spacers may be provided to ensure a certain distance (x4).

The tubular device (Fig. 2) is composed of immunoisolation layers each molded into a tubular shape (y1, y2, and y3). A cell-capturing layer (A) is enclosed inside of a tubular portion (y4) and heat, ultrasound, high frequency, electron beam, or the like is applied to seal both ends of the tubes by fusing, thus molding a tubular device.

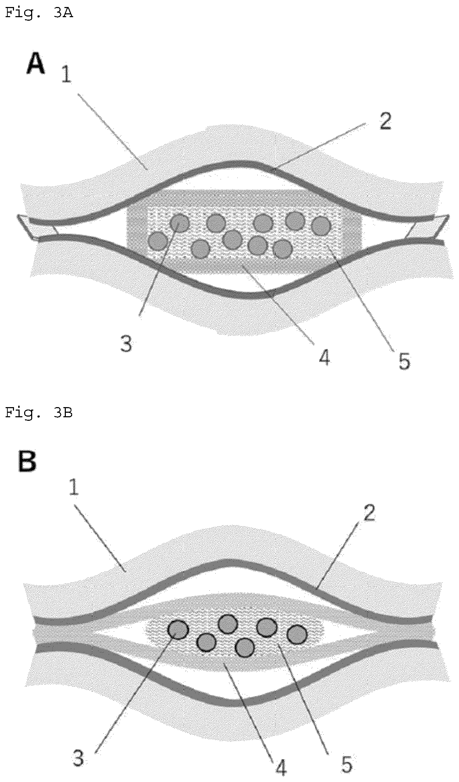

Figs. 3A and 3B are conceptual diagrams of immunoisolation devices according to one embodiment of the present invention. In Figs. 3A and 3B, the outermost layer of the immunoisolation layer (B) is in contact with the transplantation site, whereas the innermost layer is in contact with the cell-capturing layer (A).

As shown in Figs. 3A and 3B, which are conceptual diagrams of immunoisolation devices, each immunoisolation device has a pouch-like or tubular shape, and a cell-capturing layer in which the material to be transplanted is dispersed is enclosed inside of the device. In this embodiment, it is shown that the cell-capturing layer (A) contains a material to be transplanted, such as cells and a cell cluster, in such a manner that the material to be transplanted is uniformly dispersed and fixed to the fiber structure (a1) (5) that is present inside of the cell-capturing layer (A). The fiber structure (a1) (5) preferably contains at least one member selected from the group consisting of ethylene-vinyl alcohol copolymers and cellulose acetate, which have excellent biocompatibility, or may be a cell scaffold material, such as collagen fibers.

As the fiber structure (a1) (5), any sterilized one can be used. The fiber structure (a1) (5) may be present in the cell-capturing layer (A) surrounded by the immunoisolation layer (B) before introducing the material to be transplanted into the cell-capturing layer (A), or the material to be transplanted (3) and the fiber structure (a1) (5) may be introduced later as the cell-capturing layer (A) into the immunoisolation device composed of the immunoisolation layer (B).

Further, when the material to be transplanted is dispersed in the fiber structure (a1) that constitutes the cell-capturing layer (A), polyvinyl alcohol or polyethylene glycol-based hydrogel particles, blocked structures, etc. may be suspended with the material to be transplanted so as to prevent aggregation or association between the cells or cell clusters that are the material to be transplanted.

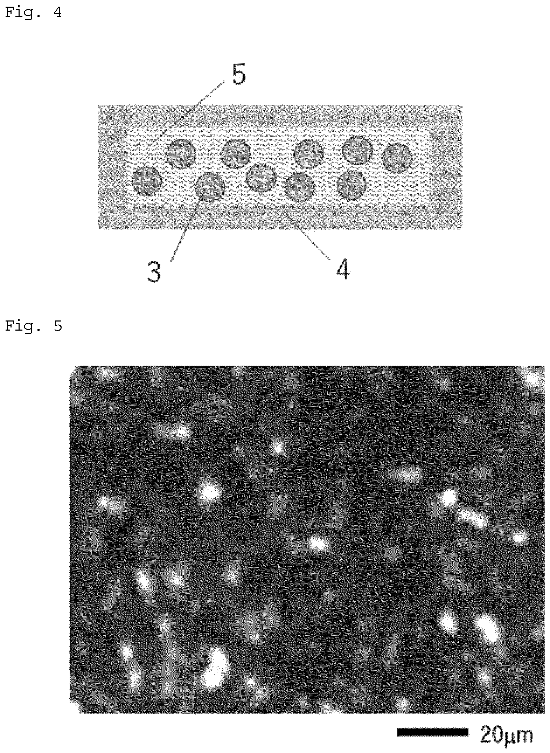

Fig. 4 is a conceptual diagram of the cell-capturing layer (A). The cell-capturing layer (A) preferably comprises two types of components, i.e., a fiber structure (a1) and a dense fiber structure (a2), and has a structure in which the dense fiber structure (a2) having smaller pores is disposed around the fiber structure (a1) for capturing the material to be transplanted to cover the fiber structure (a1) with the dense fiber structure (a2) so as to prevent the captured material to be transplanted from escape from the fiber structure.

Fig. 5 is a transmitted light microscopic image of the dense fiber structure (a2). Images such as the one shown in Fig. 5 are acquired and the area of each bright spot through which light transmits is measured using image analysis software. The diameter calculated with the assumption that the obtained area is an area of a perfect circle is taken as the pore diameter of the bright spot. The average of the pore diameters of 50 or more bright spots is defined as the average pore diameter.

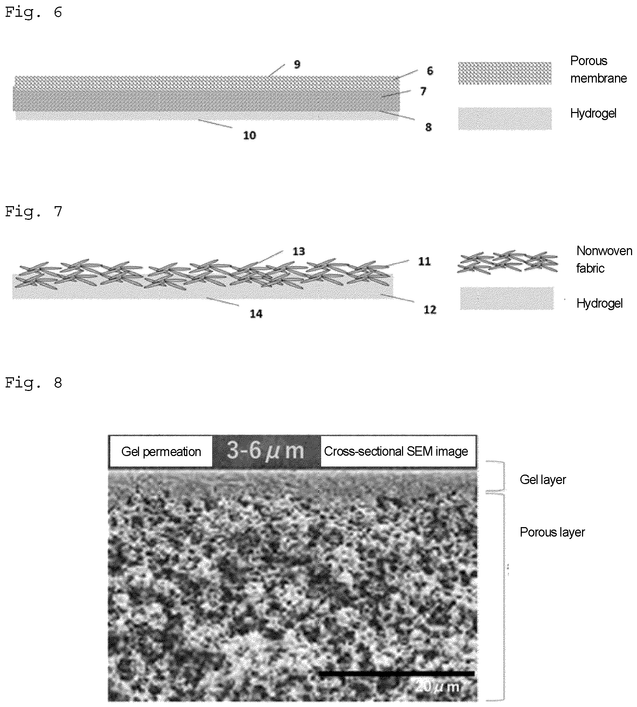

The immunoisolation layers (B) shown in Figs. 6 and 7 are composed of a combination of multiple materials. The outermost layer is to be in contact with the recipient's tissue at the transplantation site, and the innermost layer is to be in contact with a cell-capturing layer (A). Such an immunoisolation layer is formed into a pouch-like shape (Fig. 1) or a tubular shape (Fig. 2), and a cell-capturing layer (A) having fixed thereto cells or a cell cluster, which is a material to be transplanted, is enclosed therein, thus producing an immunoisolation device.

Fig. 6 shows the formation of a multilayer of a porous membrane (b1) (6) and a hydrogel (b3) (8). The porous membrane (b1) (6) is impregnated with the hydrogel (b3) to form a multilayer (7), wherein the outermost layer surface (9) is composed of a porous membrane (b1) and the innermost layer surface (10) is composed of a hydrogel (b3).

Fig. 7 shows the formation of a multilayer of a fiber structure (b2) (11) and a hydrogel (b3) (12). The hydrogel (b3) (12) is applied to the fiber structure (b2) (11) or the fiber structure (b2) (11) is impregnated with the hydrogel (b3) (12) to form a multilayer, wherein the outermost layer surface (13) is composed of the fiber structure (b2), and the innermost layer surface (14) is composed of the hydrogel (b3).

Examples

Production of Immunoisolation Multilayer (B')

Production Example 1

A porous membrane formed using an ethylene-vinyl alcohol copolymer (referred to below as EVOH) and a hydrogel containing a methacryloyl-modified polyvinyl alcohol (referred to below as MA-PVA) as a main component were laminated to form a multilayer by the following procedure.

(1) A porous membrane (average pore size: 1.8 $\mu$m, maximum pore size: 3.4 $\mu$m, thickness: 100 um) was produced by subjecting EVOH (EVAL (registered trademark), F101A, produced by Kuraray Co., Ltd.) to a polymer phase separation reaction.

(2) Lithium phenyl(2,4,6-trimethylbenzoyl)phosphinate was added as a water-soluble photoradical polymerization initiator to a 10 mass% aqueous solution of MA-PVA (average degree of polymerization: 1700, saponification degree: 98.0 to 99.0 mol%, methacryloyl modification rate: 1.2 mol%; referred to below as MA-PVA) to achieve a concentration of 0.1 mass%, thus preparing a sol.

(3) This sol was applied to a PET film to a thickness of 50 $\mu$m using a bar coater.

(4) The porous membrane was placed on the sol, and the sol and the porous membrane were tightly adhered to each other using a laminator.

(5) The sol surface was placed on top, and irradiation was performed with 365 nm light at an intensity of 15 mW/cm$^2$ for 3 minutes to form a hydrogel on the porous membrane, thus obtaining an immunoisolation multilayer (B'). Fig. 8 shows an SEM image of a cross-section of the obtained immunoisolation multilayer (B'). The hydrogel was found to have permeated the porous membrane to a depth of about 3 to 6 $\mu$m.

Production Example 2

Using a 4 mass% aqueous solution of MA-PVA (average degree of polymerization: 3500, saponification degree: 87.0 to 89.0 mol%, methacryloyl modification rate: 1.2 mol%), a hydrogel was formed on a porous membrane in the same manner as in Production Example 1 to prepare an immunoisolation multilayer (B').

Material Permeability Test

Permeabilities of the glucose, insulin, and IgG of the porous membrane produced in Production Example 1 and the immunoisolation multilayers (B') obtained in Production Example 1 or 2 were measured in the following manner.

(1) The porous membrane or the immunoisolation multilayer were each placed between chambers a and b (Fig. 9). An aqueous solution (65 mL) containing insulin (30 U/L), glucose (5 mg/mL), and IgG (0.5 $\mu$g/mL) was prepared in chamber a, whereas water (65 mL) was placed in chamber b.

(2) Stirring was performed with a stirrer at a constant temperature of 37°C. 20 hours later, liquid was sampled from chamber b, and the concentration changes of insulin, glucose, and IgG were measured by ELISA.

(3) The permeability of each substance was calculated from the results of (2) (Table 1).

Permeability (%) of each substance = Concentration of each substance in chamber b after 20 hours of measurement / (Concentration of each substance in chamber a at the start of measurement ÷ 2) $\times$ 100

Table 1

| | | Permeability after 20 hours (%) | | |
|---|---|---|---|---|
| | | Glucose | Insulin | IgG |
| Reference Example 1 | EVOH porous membrane | 72 | 25 | 3 |
| Production Example 2 | Immunoisolation multilayer | 60 | 8 | 0 |
| Production Example 3 | Immunoisolation multilayer | 69 | 17 | 0 |

Preparation of Cell-capturing Layer

Production Example 3

Preparation of Fiber Structure (a1)

(1) A core-sheath composite fiber (fineness: 3.3 dtex, specific gravity: 1.25, trade name: "Sophista" S-220, produced by Kuraray Co., Ltd., ethylene content of ethylene-vinyl alcohol copolymer: 44 mol%, saponification degree: 98.4 mol%) comprising a polyester as the core material and an ethylene vinyl alcohol copolymer as the sheath material was used to produce a fiber sheet called a web by the card method.
(2) The web was molded with the web being interposed between a pair of conveyor rollers.
(3) Subsequently, the molded web was subjected to steam jet to bond fibers together by heat and jet stream of the steam, thus obtaining a nonwoven fabric sheet with a basis weight of 50 g/m$^2$ and a thickness of 1 mm.
(4) A fiber structure for capturing cells (a1) (porosity: 96%) was prepared by cutting the nonwoven fabric sheet into circular shape using a hole punch with a diameter of 15 mm. The porosity was calculated from the basis weight C (g/m$^2$) of the fiber structure, thickness D (cm), and average specific gravity E (g/cm$^2$) of the fiber according to the following formula:

$$\text{Porosity (\%)} = 100 - ((C/D/E) \times 10^{-4} \times 100)$$

Production Example 4

Preparation of Dense Fiber Structure (a2)

(1) Using a general meltblown facility, a molten ethylene-vinyl alcohol copolymer (ethylene content: 44 mol%, saponification degree: 99.3 mol%, "E105," produced by Kuraray Co., Ltd.) was extruded from a die to obtain a meltblown nonwoven fabric sheet having a fiber diameter of 8 $\mu$m.
(2) The meltblown nonwoven fabric sheet was calendered by passing the sheet through 100°C-heated metal rolls and rubber rolls at a pressure of 40 kg/cm and a speed of 3 m/min. The meltblown nonwoven fabric obtained after the calendering process had a basis weight of 76.6 g/m$^2$, a thickness of 0.24 mm, and a porosity of 73%. The porosity was calculated from the basis weight C (g/m$^2$) of the dense fiber structure, thickness D (cm), and the average specific gravity E (g/cm$^2$) of the fiber structure according to the following formula:

$$\text{Porosity (\%)} = 100 - ((C/D/E) \times 10^{-4} \times 100)$$

(3) The meltblown nonwoven fabric was cut into a 24 mm square to prepare a dense fiber structure for preventing the escape of cells (a2) (average pore size: 11 $\mu$m). The area of each bright spot through which light transmitted was measured from a transmitted light microscopic image (Fig. 5) of the dense fiber structure (a2) using image analysis software. The average diameter (of 50 spots) calculated with the assumption that the measured area was an area of a perfect circle was defined as the average pore diameter.

Cell Preparation

Production Example 5

MIN6 (mouse pancreatic islet-like cell line) was used to confirm the cell- and cell cluster-capturing function of the cell-capturing layer. MIN6 was seeded at a density of $2.8 \times 10^5$ cells/cm$^2$ in a suspension cell culture flask (produced by Sumitomo Bakelite Co., Ltd.) containing DMEM medium, and cultured at 37°C for 7 days to produce a cell cluster with a diameter of about 100 to 200 $\mu$m. 1 mL of a cell suspension was collected from the flask and treated with 1 mL of trypsin-EDTA to disperse the cell cluster into individual cells, and the cell density was measured using a hemocytometer. Based on the measured cell density, when cells were seeded in the following Examples, the amount of liquid was adjusted so that $5 \times 10^5$ cells were seeded in the fiber structure and subjected to testing.

Production and Evaluation of Immunoisolation Devices

Example 1

1) Seeding of Cells and Enclosure of the Cells into Immunoisolation Device

(1) The immunoisolation multilayer (B') prepared in Preparation Example 2 was cut into a 24 mm square and placed with the hydrogel surface side facing up. The dense fiber structure (a2) produced in Production Example 4 was placed on top of it, and the fiber structure (a1) produced in Example 3 was further placed at the center on top of it.
(2) Subsequently, the MIN6 cell cluster suspension produced in Production Example 5 (containing $5 \times 10^5$ cells in 50 $\mu$l of DMEM medium) was seeded onto the fiber structure (a1) from the top using a micropipette and allowed to stand for 30 seconds to allow the cell cluster suspension to permeate the fiber structure (a1).
(3) The dense fiber structure (a2) produced in Production Example 4 was placed on top of the fiber structure (a1) in which the cells had been seeded, and then the immunoisolation multilayer (B') produced in Production Example 2 was placed on top with the hydrogel surface side facing down. The four edges of the overlapped immunoisolation layer (B') and the dense fiber structure (a2) were fused together using a heat sealer (Clip Sealer Z-1, produced by Technoimpulse Co., Ltd.) to produce an immunoisolation device (Fig. 3B).

2) Assessment of Cell Viability

The immunoisolation device produced in the previous section was placed in a 6-well plate, 7 mL of DMEM medium was added, and the device was cultured at 37°C for 1 week. Half of the medium (3.5 mL) was replaced every day. As a comparative control, 7 mL of DMEM medium was added to a 6-well plate with an ultra-low adhesion surface (produced by Corning), and the cell cluster suspension prepared in the previous section was added to the 6-well plate in an amount in terms of the number of cells equal to the amount in the test device and cultured at 37°C for 1 week while half the amount (3.5 mL) of the medium was replaced every day.
After 1 week of culture, the medium was completely removed. After 1 mL of fresh medium was added, 1 mL of ATP activity assay reagent (CellTiter-Glo (registered trademark), produced by Promega K.K.) was added. The plate was shaken using a plate shaker for 2 minutes. The mixture was then allowed to stand at room temperature for 10 minutes, and 100 $\mu$L of the supernatant was sampled and transferred to a white 96-well plate. The luminescence intensity was measured using a plate reader (ARVO X2; produced by PerkinElmer). As a result, when the luminescence intensity obtained from the control cell cluster was defined as a viability of 100%, the luminescence intensity obtained from the cell cluster enclosed in the device showed a viability of 103%.

Example 2

An immunoisolation device was produced in the same manner as in Example 1, except that the basis weight of the fiber structure (a1) was changed to 100 g/m$^2$ (porosity: 92%). The cell viability was measured in the same manner as in Example 1 and was found to be 88%.

Example 3

An immunoisolation device was produced in the same manner as in Example 1, except that the basis weight of the fiber structure (a1) was changed to 25 g/m$^2$ and the thickness was changed to 0.5 mm (porosity: 96%). The cell viability was measured in the same manner as in Example 1 and was found to be 103%.

Example 4

An immunoisolation device was produced in the same manner as in Example 1, except that the basis weight of the dense fiber structure (a2) was changed to 22.3 g/m$^2$ and the thickness was changed to 0.12 mm (average pore size 23.5 $\mu$m, porosity 84%). The cell viability was measured in the same manner as in Example 1 and was found to be 96%.

Table 2

| | Cell-capturing layer (A) | | | | | | | | Immunoisolation multilayer (B') | | Cell viability (%) |
| | Fiber structure (a1) | | | | Dense fiber structure (a2) | | | | Outermost layer | Innermost layer | |
| | Basis weight (g/m$^2$) | Thickness (mm) | Density (g/cm$^3$) | Porosity (%) | Basis weight (g/m$^2$) | Thickness (mm) | Average pore size ($\mu$m) | Porosity (%) | | | |
|---|---|---|---|---|---|---|---|---|---|---|---|
| Example 1 | 50 | 1 | 0.05 | 96 | 76.6 | 0.24 | 11 | 73 | EVOH porous membrane | Hydrogel | 103 |
| Example 2 | 100 | 1 | 0.1 | 92 | 76.6 | 0.24 | 11 | 73 | EVOH porous membrane | Hydrogel | 88 |
| Example 3 | 25 | 0.5 | 0.05 | 96 | 76.6 | 0.24 | 11 | 73 | EVOH porous membrane | Hydrogel | 103 |
| Example 4 | 50 | 1 | 0.05 | 96 | 22.3 | 0.12 | 23.5 | 84 | EVOH porous membrane | Hydrogel | 96 |

As is clear from the results in Table 2, in Examples 1 to 4, a cell cluster could be captured without the escape of the cell cluster from the cell-capturing layer (A).

Further, the fiber structure (a1) having an appropriate porosity can capture the cell cluster while maintaining a high viability. Furthermore, the dense fiber structure (a2) having an appropriate average pore size can prevent the cell cluster from escaping from the fiber structure (a1).

Industrial Applicability

The present invention relates to a transplantation device for use in cell transplantation therapy and the like, and in particular to an immunoisolation device for protecting materials to be transplanted from immune rejection reactions.

The immunoisolation device is assumed to be mainly used as a regenerative medicine product for cell transplantation therapy. However, the immunoisolation device can also be applied to the transplantation of physiologically active substances other than cells, such as enzymes, hormones, and drugs.

Explanation of Reference Symbols

x1     Immunoisolation layer (B)
x2     Immunoisolation layer (B)
x3     Fusing
x4     Certain distance
y1     Immunoisolation layer (B)
y2     Immunoisolation layer (B)
y3     Immunoisolation layer (B)
y4     Tubular interior
1     Porous membrane (b1)
2     Hydrogel (b3)
3     Material to be transplanted
4     Dense fiber structure (a2)
5     Fiber structure (a1)
6     Porous membrane (b1)
7     Impregnation and Formation
8     Hydrogel (b3)
9     Outermost layer surface
10     Innermost layer surface
11     Fiber structure (b2)
12     Hydrogel (b3)
13     Outermost layer surface
14     Innermost layer surface

**Claims**

1. An immunoisolation device comprising

   a cell-capturing layer (A) and
   an immunoisolation layer (B) that covers the cell-capturing layer (A),
   the cell-capturing layer (A) comprising a fiber structure (a1).

2. The immunoisolation device according to claim 1, wherein the fiber structure (a1) has a porosity of 90% or more.

3. The immunoisolation device according to claim 1 or 2, wherein the fiber structure (a1) has a thickness of 100 to 2000 $\mu$m.

4. The immunoisolation device according to any one of claims 1 to 3, wherein the fiber structure (a1) comprises at least one member selected from the group consisting of ethylene-vinyl alcohol copolymers and cellulose acetate.

5. The immunoisolation device according to any one of claims 1 to 4, wherein

the cell-capturing layer (A) comprises the fiber structure (a1) and a dense fiber structure (a2), and
the dense fiber structure (a2) is disposed around the fiber structure (a1) and has an average pore size of 35 μm or less.

6. The immunoisolation device according to claim 5, wherein the dense fiber structure (a2) has a thickness of 1500 μm or less.

7. The immunoisolation device according to claim 5 or 6, wherein the dense fiber structure (a2) comprises at least one member selected from the group consisting of ethylene-vinyl alcohol copolymers and cellulose acetate.

8. The immunoisolation device according to claim 1, wherein

the cell-capturing layer (A) comprises a dense fiber structure (a2) disposed around the fiber structure (a1) and is capable of preventing escape of cells or a cell cluster captured in the fiber structure (a1), and
the dense fiber structure (a2) has the function of preventing escape of the cell or the cell cluster.

9. The immunoisolation device according to any one of claims 1 to 8, wherein

the immunoisolation layer (B) comprises a porous membrane (b1) or a fiber structure (b2), and
the porous membrane (b1) or the fiber structure (b2) comprises at least one member selected from the group consisting of ethylene-vinyl alcohol copolymers and cellulose acetate.

10. The immunoisolation device according to claim 9, wherein the immunoisolation layer (B) is an immunoisolation multilayer (B') comprising the porous membrane (b1) or the fiber structure (b2), and a hydrogel (b3).

11. The immunoisolation device according to claim 10, wherein the immunoisolation multilayer (B') has a thickness of 500 μm or less.

12. The immunoisolation device according to claim 10 or 11, wherein the outermost layer of the immunoisolation multilayer (B') is the porous membrane (b1) or the fiber structure (b2), and the innermost layer is the hydrogel (b3).

13. The immunoisolation device according to claim 10 or 11, wherein

the outermost layer of the immunoisolation multilayer (B') is the hydrogel (b3), and
the innermost layer is the porous membrane (b1) or the fiber structure (b2).

14. The immunoisolation device according to any one of claims 10 to 13, wherein

the hydrogel (b3) comprises a polyvinyl alcohol, and
the average degree of polymerization of the polyvinyl alcohol is 300 to 10000.

15. A method for producing an immunoisolation device comprising a cell-capturing layer (A) that comprises a fiber structure (a1) and an immunoisolation multilayer (B') that covers the cell-capturing layer (A),
the method comprising the steps of:

(1) applying a hydrosol solution to a porous membrane (b1);
(2) converting the hydrosol solution into a hydrogel by heat, temperature, light, or chemical action to form an immunoisolation multilayer (B'); and
(3) molding the immunoisolation multilayer (B') into a pouch shape by heat fusion.

16. The method for producing an immunoisolation device according to claim 15, wherein

the hydrosol solution in step (1) comprises a polyvinyl alcohol,
the polyvinyl alcohol has a degree of polymerization of 300 to 10000, and
the hydrosol solution has a solids concentration of 3 to 15 mass%.

17. The method for producing an immunoisolation device according to claim 15 or 16, wherein

the heat fusion in step (3) is performed with a resin being interposed between two sheets of the immunoisolation multilayer (B'),
the two sheets of the immunoisolation multilayer (B') are disposed with hydrogel surfaces facing each other, and
the resin comprises at least one member selected from the group consisting of ethylene-vinyl alcohol copolymers and cellulose acetate.

Fig. 1

Fig. 2

Fig. 3A

Fig. 3B

Fig. 4

Fig. 5

20μm

Fig. 6

| | Porous membrane |
| | Hydrogel |

Fig. 7

| | Nonwoven fabric |
| | Hydrogel |

Fig. 8

Fig. 9

Test substance

a    b

## INTERNATIONAL SEARCH REPORT

International application No.

**PCT/JP2023/016713**

### A.   CLASSIFICATION OF SUBJECT MATTER

***A61L 27/16***(2006.01)i; ***A61L 27/20***(2006.01)i; ***A61L 27/52***(2006.01)i
FI:   A61L27/16; A61L27/20; A61L27/52

According to International Patent Classification (IPC) or to both national classification and IPC

### B.   FIELDS SEARCHED

Minimum documentation searched (classification system followed by classification symbols)

A61L27/16; A61L27/20; A61L27/52

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C.   DOCUMENTS CONSIDERED TO BE RELEVANT

| Category* | Citation of document, with indication, where appropriate, of the relevant passages | Relevant to claim No. |
|---|---|---|
| Y | JP 2021-3527 A (HITACHI, LTD.) 14 January 2021 (2021-01-14)<br>claims 1, 6, 7, paragraphs [0025], [0026], fig. 2 | 1-17 |
| Y | JP 2021-500160 A (EMBODY INC.) 07 January 2021 (2021-01-07)<br>claims 1, 5, paragraph [0135] | 1-17 |
| Y | JP 2016-524967 A (INST. NATIONAL DE LA SANTE ET DE LA RECHERCHE MEDICALE) 22 August 2016 (2016-08-22)<br>claims 1, 8, 15 | 1-17 |
| Y | JP 9-503941 A (THE REGENTS OF THE UNIV. OF MICHIGAN) 22 April 1997 (1997-04-22)<br>claim 9, page 24 | 4-7, 9-14, 17 |

☐ Further documents are listed in the continuation of Box C.         ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **07 July 2023** | **18 July 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
### Information on patent family members

International application No.

**PCT/JP2023/016713**

| Patent document cited in search report | | | Publication date (day/month/year) | Patent family member(s) | Publication date (day/month/year) |
|---|---|---|---|---|---|
| JP | 2021-3527 | A | 14 January 2021 | (Family: none) | |
| JP | 2021-500160 | A | 07 January 2021 | US 2022/0176017 A1 claims 1, 5, paragraph [0179] WO 2019/084209 A1 claims 1, 5, paragraph [0197] EP 3700462 A1 claims 1, 5, paragraph [193] | |
| JP | 2016-524967 | A | 22 August 2016 | US 2016/0136330 A1 claims 1, 8, 15 WO 2015/007797 A1 claims 1, 8, 15 EP 3021880 A1 claims 1, 8, 15 | |
| JP | 9-503941 | A | 22 April 1997 | US 5686289 A page 9, lines 29, 30 WO 1995/011048 A2 claim 8, paragraph [0047] | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012508584 A